# EUROPEAN PATENT APPLICATION

(11) **EP 2 740 466 A1**
(43) Date of publication of application: **11.06.2014**
(21) Application number: 12196268.2
(22) Date of filing: 10.12.2012
(51) Int. Cl.: A61K 8/34, A61K 8/41, A61Q 5/12, A61K 8/898

(54) **Pearlescent hair care composition**

(71) Applicant: OTC GmbH, 46047 Oberhausen (DE)
(72) Inventor: Dahms, Gerd, 47138 Duisburg (DE)
(74) Representative: Michalski Hüttermann & Partner Patentanwälte

(57) **Abstract**

The present invention relates to a pumpable or sprayable pearly luster hair care composition with a high humectant content and high heat stability, the use of such cosmetic hair care composition and a kit of parts containing said pearly luster hair care composition.

## Description

### FIELD OF THE INVENTION

The present invention relates to a pumpable or sprayable pearly luster hair care composition with a high humectant content and high heat stability, the use of such cosmetic hair care composition and a kit of parts containing said pearly luster hair care composition.

### BACKGROUND OF THE INVENTION

The formulation strategy of cosmetic products includes, besides pure functional characteristics, also the aesthetic appeal, which is highly relevant and one crucial parameter guiding the customer in his buying decision. This fact is taken into account by the industry especially with respect to the choice of the packaging and the design of the product optical appearance. Here coloring substances are mainly used, which are either able to simply tint the composition or to induce more sophisticated optical outcomes. In the latter group effect pigments can be found, which for instance are able to induce a sparkling effect, change the color as a function of the incidence angle or provide pearly luster. In particular pearly luster effect substances, which provide a silky or mother-of-pearl-like appearance to cosmetic composition, enhance the products aesthetics and are widely used in body care and cleansing products like shampoo or hair conditioner. The pearl luster effect in an aqueous liquid or dispersion is usually achieved by addition of inorganic pigments or organic substances, which both are able to form special OD 41687 / UAM supra-molecular structures in the presence of emulsifier. The interaction of the light with the supra-molecular structures of the pearly luster components is the reason for the pearly luster effect.

Several different pearl-luster compounds and pearl-luster compositions in cosmetics have been described in the literature. For instance, EP 0846 752 A2 describes pearl-luster dispersions containing the following components: (A) 5-30 weight-% behenic acid (salts) ; (B1) 1-50 weight-% one or more compounds of formula R₁-O-(C₂H₄O)ₙ-SO₃⁻ X⁺ (I) ; or (B2) 1-50 weight-% one or more compounds of formula R₂-COO-CH₂-CH₂-SO₃⁻ X⁺ (II) ; or (B3) 1-50 weight-% mixture of (I) and (II) ; and (C) the balance water and optional standard components, wherein R₁ = 12-14C alkyl; R₂ = 8-18C alkyl; n = 2-3 ; and X = alkali metal or ammonium. In addition also surfactant formulations containing 1-10 weight-% of these dispersions are also claimed.

In another approach EP 1771 144 discloses cosmetic preparations, particularly hair conditioners, comprising at least one quaternary ammonium compound, at least one amino-functional silicone, and at least one poly-hydroxy compound with at least 2 OH groups. The weight ratio between amino-functional silicones and poly-hydroxy compounds ranges between 1:1 and 100:1.

Furthermore, WO 92/13512 suggests lustrous concentrates containing (A) 15-40 % weight-% lustrous components, (B) 5-55 % weight-% emulsifiers and (C) 15-40 % weight-%. low-molecular polyvalent alcohols which can be poured or pumped and can be stored for long periods without the addition of preserving agents.

Nevertheless, one drawback of the standard pearly-luster compositions is that usually the effect is a strict function of the presence of the supra-molecular structures. As a rule mechanical forces, for instance generated in the course of the application via pumping or spraying, or heat are disturbing or destroying such structures, resulting in a reduced pearly-luster effect in the composition. Such kind of behavior is disadvantageous, because it restricts the amount of available application devices and requires a stringent temperature control during storage and processing.

### SUMMARY OF THE INVENTION

The present invention has the object of providing an improved pumpable or sprayable pearly luster hair care composition with a high humectant content and high heat stability, the use of such cosmetic hair care composition and a kit of parts containing said pearly luster hair care composition. This object is achieved by a composition according to claim 1. Preferred embodiments of the invention are disclosed in the dependent claims.

### DETAILED DESCRIPTION OF THE PREFERRED EMBODIMENTS

This object is achieved according to the invention by the provision of a heat stable, pearly luster hair care composition, characterized in that the compositions comprises
a) ≥ 10 weight-% and ≤ 95 weight-% of at least one low molecular weight polyol,
b) ≥ 0.5 weight-% and ≤ 20 weight-% of at least one quaternary mono-alkyl-trimethyl- or di-alkyl-dimethyl-ammonium salt,
c) ≥ 1 weight-% and ≤ 30 weight-% of at least one ethoxylated amino functional silicon derivative,
d) ≥ 0 weight-% and ≤ 10 weight-% of at least one cosmetic or therapeutic active,
e) ≥ 0 weight-% and ≤ 10 weight-% of at least one auxiliary substance and
f) ≥ 0 weight-% and ≤ 78.5 weight-% water,
wherein the components a) to f) add to 100 weight-%.

Surprisingly it has been found that such an inventive hair care composition provides a pearly luster effect, which is stable even after pumping or spraying operations and can even be heated to high temperatures without losing the optical properties of the composition. Once the pearly luster effect has formed in the composition by mixing of the ingredients, the optical luster effect is stable and the composition can be used as a concentrate and be diluted by conventional means without losing the pearly luster appearance. Within or after the processing the composition can be heated up to temperatures up to 60°C without reduction of the optical effect. It is also especially surprising that this effect can be achieved without the presence of fatty acid alcohols, which are usually introduced to induce or stabilize the pearly luster effect. Without being bound by the theory the pearly luster in the inventive composition is achieved by lamellar structures which are formed by the quaternary ammonium salts and/or ethoxylated amino functional silicon derivatives. It appears that the low molecular weight polyol is able to stabilize these structures and also contributes to the exceptional high heat stability and the good mechanical stability of the pearly luster effect. In addition, the inventive high concentration of the low molecular weight polyol contributes to the care effect of the composition. Especially the water binding capacity and the penetration behavior of such substances render them very suitable in the inventive hair care treatment. Besides the application of the composition in the course of a hair care cosmetic treatment also skin care application may be possible. No restrictive elements or composition essentials can be seen which render a cosmetic skin care not within the scope of this invention.

A hair care composition in the sense of this invention is a liquid composition which can be used as a product in any kind of hair care treatment, i.e a shampoo, hair rinse, mousse, pre-shampoo, hair spray, hair brushing lotion, hair setting lotion, hair liquid, hair tonic and the like. The hair care treatment can be designed as a leave on or a rinse-off treatment. Within a leave-on treatment the composition is applied and is left without removal. In a rinse-off treatment the composition is applied to the hair and removed after a certain application time, which is usually in a timescale off a few minutes up to an hour.

Surprisingly it has been found that especially the use of a low molecular weight polyol in a concentration range of ≥ 10 weight-% and ≤ 95 weight-% in the composition yields a very brilliant pearly luster effect, enhances the stability of the of the pearly luster effect and contributes to the cosmetic efficacy of the composition. A low molecular weight polyol in the sense of this invention comprises a molecular weight below 500 g/mol, preferably below 400 g/mol and more preferably below 300 g/mol and more than one hydroxyl moiety. Such kinds of polyols have the right size to penetrate within the application time into the skin or hair and hence contribute to the cosmetic efficacy and, in addition, they are able to stabilize and enhance the pearly-luster effect. Mono-ols in such a high concentration in the composition are not suitable with respect to the cosmetic efficacy and furthermore they might interfere with or inhibit the pearly luster effect.

The at least one quaternary mono-alkyl trimethyl- or di-alkyl-dimethyl-ammonium salt is selected from the group comprising quaternary mono-alkyl-trimethyl- or di-alkyl-dimethyl-ammonium salts and is represented by formula 1: 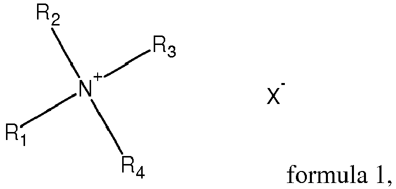 wherein X = Cl or Br; R₁ = Methyl; R₂ = C8-C25 n- or iso-alkyl, preferably C18-C22-n-alkyl; R₃ = R₁ or R₂; R₄ = R₁.

Such kind of substances are usually employed as cationic polymers or cationic surfactants and surprisingly it at has been found that such kind of quaternary ammonium salts are able to support the pearly luster effect of the composition. Without being bound by the theory it is assumed that this group of ammonium salts either is incorporated within the lamellar structures or that this group of ammonium salts is supporting other substances like the ethoxylated amino functional silicon derivative to built up the lamellar structure. Furthermore, such kind of quaternary ammonium salts is also able to stabilize the pearly luster effect with respect to resistance to thermal or mechanical stress. The concentration range of the quaternary ammonium salt is ≥ 0.5 weight-% and ≤ 25 weight-%, preferably ≤ 15 weight-% and more preferably ≤ 10 weight-%. At lower concentrations no effective stabilization and cosmetic efficacy in the hair care treatment can be detected. Higher concentrations lead to a destabilization of the lamellar structures and as a consequence to a reduced pearly luster effect. The concentration of the quaternary ammonium salts can be determined using standard analytical techniques like HPLC. Such kind of techniques can be employed for all concentration determinations within this composition except for the water.

Another ingredient of the inventive composition can be at least one ethoxylated amino functional silicon derivative, which is present in a concentration ≥ 1 weight-% and ≤ 30 weight-%. The derivative is a silicon-derivative comprising at least one (substituted or non-substituted) amino-moiety and is additionally ethoxylated at the silicon backbone or the amino-moiety. The cloud point of the ethoxylated amino functional silicon derivative can be in the range of 25 - 80 °C, preferably between 30 - 50 °C and more preferably between 35 - 45 °C and the degree of ethoxylation can be between 1:1 to 100:1, preferably between 1:1 to 50:1 and more preferably between 1:1 to 25:1 (mol ethylenoxid: mol aminofunctional silikon). Preferably the ethoxylated amino functional silicon derivative can be present in a concentration ≥ 2.5 weight-% and ≤ 15 weight-% and more preferably in a concentration ≥ 5 weight-% and ≤ 10 weight-%. This concentration range has proven to result in a very effective hair conditioning and ensures or stabilizes a sufficient pearly luster effect in the composition.

Within a preferred embodiment of the invention the ethoxylated amino functional silicon derivative may comprise trideceth-9 PG-amodimethicone and trideceth-12 (Silcare SEA, Clariant AG).

The cosmetic or therapeutic active is incorporated within the composition between ≥ 0 weight-% and ≤ 10 weight-%, i.e. it is also possible in certain embodiments of the invention that no further cosmetic or therapeutic active is present. Otherwise the cosmetic or therapeutic active can be selected from the group comprising hair styling actives like liquid oils and fat, wax, hydrocarbon oil, higher fatty acid, higher alcohol, ester oil, silicone oil, anti-dandruff agents, hair dyes, anionic surfactant, cationic surfactant, ampholytic surfactant, nonionic surfactant, UV absorbent, polyhydric alcohol, metal ion sequestrant, sugar, amino acid, organic amine, hair styling polymers, antimicrobial agents hair-removing agents, conditioning agents for the skin or the scalp, skin or scalp-smoothing agents, agents for increasing the hydration of the skin or the scalp, sun protection agents, keratolytics, radical acceptors for free radicals, antiseptic active ingredients, active ingredients for treating the signs of skin or scalp ageing and/or agents which modulate the differentiation and/or proliferation and/or pigmentation of the skin or the scalp, vitamins such as vitamin C, active ingredients having a stimulating side-effect, such as alpha-hydroxy acids, β-hydroxy acids, alpha-keto acids, β -keto acids, retinoids (retinol, retinal, retinic acid), anthralins (dioxyanthranol), anthranoids, peroxides (especially benzoyl peroxide), minoxidil, lithium salts, antimetabolites, vitamin D and its derivatives; catechols, flavonoids, ceramides, wheatgerm oil and vitamin E isolated therefrom, evening primrose oil, plant lecithins (e.g. soya lecithin), sphingolipids/ceramides isolated from plants, animal oils or fats, such as tallow, lanolin, fatty acid esters, esters of fatty alcohols and waxes having a melting point corresponding to the temperature of the skin (animal waxes, such as beeswax, carnauba wax and candelilla wax, mineral waxes, such as microcrystalline waxes, and synthetic waxes, such as polyethylene or silicone waxes), essential fatty acids (e.g. gamma-linolenic acid), protein hydrolyzeates, chitin, chitosan derivates, enzymes, coenzymes, enzyme inhibitors, hydrating agents, skin or the scalp -calming agents, detergents or foam-forming agents, and inorganic or synthetic delustering fillers, abrasive agents. Preferably the cosmetic or therapeutic active can be incorporated in an amount of ≥ 0.1 weight-% and ≤ 10 weight-% and even more preferably ≥ 2 weight-% and ≤ 8 weight-%.

Auxiliary substances might also be present in the composition if a special physical or chemical parameter is required in the formulation (for instance a special pH-range). Auxiliary substances in the sense of this invention may include: high molecular weight polyols, pH-adjusting agents, buffering substances, viscosity adjusting agents such as hydrocolloids including polysaccharides, homoglycans or heteroglycans, for example carragheen, pectins, tragacanth, guar gum, locust bean flour, agar-agar, gum arabic, xanthan gum, natural and modified starches, dextrans, dextrin, maltodextrins, chitosan, glucans, such as β-1,3-glucan, β-1,4-glucan, such as cellulose, mucopolysaccharides, such as especially hyaluronic acid, synthetic polymers such as cellulose ethers, polyvinyl alcohol, polyvinylpyrrolidone, synthetic cellulose derivatives, such as methylcellulose, carboxycellulose, carboxymethyl-cellulose, especially sodium carboxymethylcellulose, cellulose esters, cellulose ethers such as hydroxypropylcellulose, polyacrylic acid, polymethacrylic acid, poly(methyl methacrylate) (PMMA), polymethacrylate (PMA), polyethylene glycols" preservatives, inorganic and organic acids or bases, fatty substances, such as mineral oils, such as paraffin oils or Vaseline oils, silicone oils, vegetable oils such as coconut oil, sweet almond oil, apricot oil, corn oil, jojoba oil, olive oil, avocado oil, sesame oil, palm oil, eucalyptus oil, rosemary oil, lavender oil, pine oil, thyme oil, mint oil, cardamom oil, orange-blossom oil, soybean oil, bran oil, rice oil, rapeseed oil and castor oil, animal oils or fats, such as tallow, lanolin, butyric oil, fatty-acid esters, esters of fatty alcohols such as triglycerides, and waxes with a melting point corresponding to skin temperature (animal waxes such as beeswax, carnauba wax and candelilla wax, mineral waxes, such as microcristalline waxes, and synthetic waxes, such as polyethylene waxes or silicone waxes), as well as ail oils that are suitable for cosmetic purposes (socalled cosmetic oils), such as, for example, those mentioned in the CFTA treatise entitled Cosmetic Ingredient Handbook, 1st edition, 1988. The Cosmetic, Toiletry and Fragrance Association, Inc., Washington, cosolvents, pharmaceutically and cosmetically commonly used or other colorants and pigments, in particular those that are used primarily for the color design of the composition and not for application and color design on the human body, such as those pigments and colorants as those decorative colorants, softening agents and/or lubricants.

In a certain aspect of the invention auxiliary substances may be present in the composition comprising high molecular weight polyols. The high molecular weight polyols may exhibit a molecular weight ≥ 500 g/mol and ≤ 30 000 g/mol. Such high molecular weight polyols has been shown to contribute beneficial to the foam stability and at the same time can increase the product viscosity. One example for suitable high molecular weight polyols as auxiliary substance in the sense of the invention are Polyethylenglycols (PEGs).

The water content of the composition can be between ≥ 0 weight-% and ≤ 78.5 weight-%. Low water contents are especially suitable if a composition is required which exhibits self preserving properties. Here no additional preservative has to be added. Composition comprising a low water content might also be considered as concentrates, which can be adjusted with respect to a suitable viscosity or active range right before the application by adding water. This is possible without losing the pearly luster effect of the composition. For the dilution any water like tap-water can in principle be used, whereas de-mineralized water or distilled water is preferred.

Within a further object of the invention the low molecular weight polyol a) is selected from the group comprising branched or linear C2-C6 hydrocarbons comprising 2 - 6 hydroxyl groups. Especially low molecular weight compounds with multiple hydroxyl functional groups may contribute to the luster stability and the beneficial hair care properties of the inventive formulation. Polyols with longer hydrocarbon chain-length might interfere with the luster formation and stability and are less suitable for skin and hair hydration properties. In a preferred embodiment the polyols are non-cyclic, linear and unbranched sugar-alcohols and can be selected from the group of sugar alcohols (also known as polyhydric alcohols or glycitols). For use in the inventive composition these polyols might be polyols like glycerin, di- or tri-ethylenglycol, butylenglycol, 1, 2- or 1,3 Propylenglycol, pentamethylenglycol, erythritol, threitol, arabitol, xylitol, ribitol, mannitol, sorbitol, galactitol and iditol. The use of glycerin, butylenglycol, propylenglycol and/or pentandiol is preferred. Also the mixture of more than one low molecular weight polyol as components a) is within the scope of this invention.

In another aspect of the invention the at least one low molecular weight polyol a) is glycerin. Especially glycerin yields a very stable and distinct pearly luster effect in the composition. In addition, the cosmetic efficacy of the composition with respect to humidification of the hair and the skin/scalp are increased using glycerin as low molecular weight polyol.

In another preferred embodiment the low molecular weight polyol or the low molecular weight polyols a) are present in a concentration of ≥ 50 weight-% and ≤ 95 weight-%. Such high low molecular weight polyol concentration yield very brilliant pearly luster compositions. In another embodiment of the invention the low molecular weight polyol can preferably be present in a concentration range of ≥ 60 weight-% and ≤ 95 weight-% and even a low molecular weight polyol concentration from of ≥ 70 weight-% and ≤ 95 weight-% yield stable pearly luster compositions. Such kind of compositions with very high polyol content may be very suitable for the use as a concentrate, which can be diluted directly prior to application.

In an additional characteristic of the invention the composition can essentially be free of glycol esters of fatty acids. Glycol esters of fatty acids may disturb the protective lipid mantle of the skin, scalp and/or hair and might therefore be disadvantageous in an effective hair care treatment. Examples of glycol esters of fatty acids may include mono- or di-ester of ethylene- or propylene glycol, diethylene glycol distearate, diethylene glycol monostearate, ethylene glycol monostearate, ethylene glycol saturated fatty acid diester, ethylene glycol saturated fatty acid esters, propylene glycol dioleate, propylene glycol monostearate, propylene glycol monostearate, propylene glycol tricapryl caprate.

Further a composition is within the scope of the invention, wherein the at least one quaternary mono-alkyl trimethyl- or di-alkyl-dimethyl-ammonium salt b) is selected from the group comprising Lauryltrimethylammonium, Cocoyltrimethylammonium, Cetyltrimethylammonium, Stearyltrimethylammonium, Behenyltrimethylammonium, Distearyldimethylammonium and Dilauryldimethylammonium salts. Especially such medium-range quaternary mono- or di-alkyl ammonium salts are able to provide a sufficient solubility in the composition medium and, at the same time, are able to provide a good hair conditioning effect. Longer alkyl-chains are disadvantageous, because they might not be dissolvable in the inventive composition medium.

In an additional embodiment according to the invention the composition contains at least one quaternary ammonium salt b), wherein the at least one quaternary ammonium salt b) is a Behentrimonium salt. The Behentrimonium salt might preferably be a chloride or a bromide salt and this quaternary ammonium salt is especially suited to provide an excellent hair conditioning effect and to contribute to the pearly luster stability and formation.

In an additional inventive aspect the composition comprises components b) and c), wherein the sum of the components b) and c) is ≥ 10 weight-% and ≤ 40 weight-%. More preferably the sum of the components b) and c) is ≥ 10 weight-% and ≤ 30 weight-% and even more preferably the sum of the components b) and c) in the composition is ≥ 15 weight-% and ≤ 30 weight-%. Such an amount of surface active materials might be advantageous, because a lower content might reduce the conditioning properties of the inventive composition, here especially the combability of the wet hair, whereas larger amounts of b) and c) might increase the risk of scalp irritation and hair-dryness.

In a further embodiment of the invention the composition can be formulated in a way that it is essentially free of preservatives. The high polyol content of the formulation might lead to a significantly reduced water activity in the composition, which in turn results in a self-preserving system. Preservatives in the sense of this invention are substances especially designed and introduced into cosmetic formulations in order to inhibit the growth or to kill microorganism. Commonly used preservatives are for instance parabens, formaldehyde donors, isothiazolones, benzoates, chloracetamide, triclosan and iodopropynyl butylcarbamate. Not within this group are substances which show some anti-microbial activity, but their main purpose is a different on, for instance the quats, which also show some anti-microbial function, but are mainly used for their conditioning, tensidic or emulsifying properties.

Furthermore, in one preferred aspect of the invention the composition comprises a viscosity of ≥ 10 mPas and ≤ 200 mPas. More preferred the viscosity of the inventive composition may be between ≥ 20 mPas and ≤ 175 mPas and even more preferred between ≥ 30 mPas and ≤ 150 mPas. Such a viscosity range is advantageous, because the composition can be easily applied to the hair using a pump dispenser with or without a spray-head. Lower viscosities might be disadvantageous, because in that case the composition is too thin and might run-off the hair before it is properly distributed. Higher viscosities might be disadvantageous, because then the spreading and the distribution of the composition in the hair might be too laborious. Preferably the viscosity of the composition may be adjusted, if necessary, for instance using rheological modifiers, to the above mentioned viscosity range. The composition viscosity can be determined according to methods known to the skilled in the art, for instance using a Brookfield viscometer, spindle No. 2, at a temperature of 25°C and a spindle speed of 20 rpm.

In an additional embodiment according to the invention the composition may comprise a pH between ≥ 2.0 and ≤ 7.0, preferably between ≥ 3.0 and ≤ 6.0 and more preferably between ≥ 4.0 and ≤ 6.0. Such a pH-range may be favorable because it reduces the risk of skin irritation during and after application.

In an additional characteristic of the invention the composition can be used for the treatment of human hair. The cosmetic use of the inventive hair care composition is especially suited for a hair care treatment of human hair. The formulation is particular advantageous with respect to combability, hair volume, gloss, shine, electrostatic properties and the touch of the hair.

In another preferred embodiment of the invention the composition is applied to the hair by means of a pump or a spray dispenser. Due to the fact that the inventive hair-care composition provides a pearly luster effect with improved stability against heat and mechanical forces, the composition might also be applied to the hair using a mechanical hand-driven pump or a spray dispenser without losing the overall pearly luster effect. This enlarges the amount of suitable packaging and application material and might reduce the packaging costs.

Further, it is within the scope of the invention that the composition can be washed off the hair after an application time of 1 - 45 minutes. The inventive hair-care composition is easily applied to the hair by a pump- or spray dispenser and can be spread onto the hair without difficulties. This ensures a time saving application method and, as a function of the chosen ingredient concentration, a fast onset of the cosmetic effect.

In another characteristic of the invention the composition is diluted with an additional solvent to a dry content ≥ 1 weight-% and ≤ 20 weight-% directly before the application. Pearly luster concentrates of the state of the art usually do show reduced pearly luster stability after a dilution process due to the loss of the supra-molecular structures. Surprisingly it has been found, that even after dilution of the inventive composition to very low dry contents the loss of the pearly luster effect, which can be attributed to the destruction of the supra-molecular structure is negligible. Therefore it is also possible to use the inventive composition as a concentrate, which can be diluted either directly prior the cosmetic hair-care application or even within a separate production step to very low dry contents. The dilution can for instance be performed by addition of water and thoroughly mixing. The dry content of the composition can be determined by any conventional method, which is used for the dry content determination of emulsion. One possible method is the drying of the sample using a drying oven at 160°C for 20 minutes until a constant weight is achieved.

It is further within the scope of the invention to provide a kit of parts for hair care cosmetic use comprising
a) the composition as claimed in any of the claims 1 - 10 and
b) optionally a hair shampoo and/or
c) optionally a hair rinse solution and/or
d) optionally a hair straightening composition and/or
e) optionally at least one applicator selected from the group consisting of a spatula, a glove, a syringe, a brush, and a comb.

### Examples

### Composition 1 (ready to use):

| | Substance | Substance/INCI | Weight-% |
|---|---|---|---|
| a) | polyol | glycerol | 50 |
| b) | quaternary ammonium salt | cetrimonium chloride | 15 |
| | | behentrimonium chloride | 10 |
| c) | ethoxylated amino functional silicon derivative | trideceth-9 PG-amodimethicone and trideceth-12 | 5 |
| f) | water | | 20 |
| total | | | 100 |

### Composition 2 (concentrate)

| | Substance | Substance/INCI | Weight-% |
|---|---|---|---|
| a) | polyol | glycerol | 15 |
| b) | quaternary ammonium salt | cetrimonium chloride, | 1.5 |
| | | behentrimonium chloride | 1.0 |
| c) | ethoxylated amino functional silicon derivative | trideceth-9 PG-amodimethicone and trideceth-12 | 0.5 |
| d) | cosmetic or therapeutic active | hydrolyzed keratin | 0.2 |
| e) | auxiliary substance | PEG-40 hydrogenated castor oil fragrance | 0.5 0.3 |
| f) | water | | 81 |
| total | | | 100 |

## Claims

1. A heat stable, pearly luster hair care composition, **characterized in that** the composition comprises
a) ≥ 10 weight-% and ≤ 95 weight-% of at least one low molecular weight polyol,
b) ≥ 0.5 weight-% and ≤ 25 weight-% of at least one quaternary mono-alkyl-trimethyl- or di-alkyl-dimethyl-ammonium salt,
c) ≥ 1 weight-% and ≤ 30 weight-% of at least one ethoxylated amino functional silicon derivative,
d) ≥ 0 weight-% and ≤ 10 weight-% of at least one cosmetic or therapeutic active,
e) ≥ 0 weight-% and ≤ 10 weight-% of at least one auxiliary substance and
f) ≥ 0 weight-% and ≤ 78.5 weight-% water,
wherein the components a) to f) add to 100 weight-%.

2. The composition of claim 1, wherein the at least one low molecular weight polyol a) is selected from the group comprising branched or linear C2-C6 hydrocarbons comprising 2 - 6 hydroxyl groups.

3. The composition of any of the preceding claims, wherein the at least one low molecular weight polyol a) is glycerin.

4. The composition of any of the preceding claims, wherein the low molecular weight polyol or the low molecular weight polyols a) are present in a concentration of ≥ 50 weight-% and ≤ 95 weight-%.

5. The composition of any of the preceding claims, wherein the composition is essentially free of glycol esters of fatty acids.

6. The composition of any of the preceding claims, wherein the at least one quaternary mono-alkyl-trimethyl- or di-alkyl-dimethyl-ammonium salt b) is selected from the group comprising Lauryltrimethylammonium, Cocoyltrimethylammonium, Cetyltrimethylammonium, Stearyltrimethylammonium, Behenyltrimethylammonium, Distearyldimethylammonium and Dilauryldimethylammonium salts.

7. The composition of any of the preceding claims, wherein the at least one quaternary mono-alkyl-trimethyl- or dialkyl-dimethyl-ammonium salt b) is a Behentrimonium salt.

8. The composition of any of the preceding claims, wherein the sum of the components b) and c) is ≥ 10 weight-% and ≤ 40 weight-%.

9. The composition of any of the preceding claims, wherein the composition is essentially free of preservatives.

10. The composition of any of the preceding claims, wherein the composition comprises a viscosity of ≥ 10 mPas and ≤ 200 mPas.

11. The use of the composition as claimed in one of the claims 1 - 10 for the treatment of human hair.

12. The use according to claim 11, wherein the composition is applied to the hair by means of a pump or a spray dispenser.

13. The use according to claim 11 or 12, wherein the composition is washed off the hair after an application time of 1 - 45 minutes.

14. The use according to the claims 11 - 13, wherein the composition is diluted with an additional solvent to a dry content ≥ 1 weight-% and ≤ 20 weight-% directly before the application.

15. A kit of parts for hair care cosmetic use comprising
a) the composition as claimed in any of the claims 1 - 10 and
b) optionally a hair shampoo and/or
c) optionally a hair rinse solution and/or
d) optionally a hair straightening composition and/or
e) optionally at least one applicator selected from the group consisting of a spatula, a glove, a syringe, a brush, and a comb.
